# EUROPEAN PATENT SPECIFICATION

(11) **EP 3 829 412 B1**
(45) Date of publication and mention of the grant of the patent: **19.06.2024**
(21) Application number: 19752238.6
(22) Date of filing: 01.08.2019
(51) Int. Cl.: A61B 1/005, A61B 1/008, A61B 34/00, A61B 17/00

(54) **A JOINT**
VERBINDUNG
JOINT

(30) Priority: 02.08.2018 GB 201812622
(43) Date of publication of application: 09.06.2021
(73) Proprietor: IP2IPO Innovations Limited, London N1C 4AG (GB)
(72) Inventor: YANG, Guang-Zhong, Epsom KT19 7ND (GB); HU, Yang, London N17 9FD (GB); LI, Wei, Beijing (CN)
(74) Representative: Potter Clarkson
(86) International application number: PCT/GB2019/052160
(87) International publication number: WO 2020/025960

(56) References cited:
- EP-A2- 0 764 424
- US-A1- 2005 131 279
- US-B1- 8 347 754

## Description

This invention relates to a joint, and specifically, but not exclusively, to a joint for use in a surgical device for use in minimal invasive surgery (MIS) procedures, and to a method of fabricating such a joint. The invention is particularly directed to a flexible elongated joint forming part of a device for use in endoscopic procedures. However, the joint can be used in other applications, and the method could be used to fabricate similar devices having different applications.

MIS was introduced to ameliorate postoperative scar and reduce recovery time. MIS requires making small incisions in the abdominal wall, such as laparoscopic surgery, or gaining access through a narrow natural orifice, such as the oesophagus. The main requirement for MIS is the ability to adequately observe and operate the target anatomy from access sites that are not aligned in the most direct and ergonomically optimum positions. To provide surgeons with adequate visual feedback and dexterous tissue manipulation, endoscope and other operative surgical instruments are required to have a bendable and steerable section, also called a resiliently deformable joint

A resiliently deformable joint usually needs to provide a large central lumen which is free from control tendons which would otherwise obstruct the incorporation of lighting meaning, such as glass fibre cords and electrical wires for an optical unit and a camera unit. For operative tools acting at the distal end of a large surgical instrument interior space could also help to install rinsing the suction units that may be required. Another essential property of such a resiliently deformable joint is that it is controllable with regard to its bending direction via control tendons. A pair of tendons is often used to achieve planar bending. Three of four tendons are often used to achieve spatial bending.

It is known to use one capstan to control a pair of tendons for one degree of freedom (DOF) bending. The two tendons are configured in an antagonized fashion, and the increasing of one tendon equals the decreasing of the other. Axial channels in the resiliently deformable joint are required for guiding the tendons. Depending on the principle to achieve the controllable bending, known resiliently deformable joints can be classified into two categories.

### Bending Joint

The first category is a bending joint which makes use of material elasticity. These joints are tendon-driven compliant mechanisms. The tendons pass through the channels inside the bendable portion and terminate at the distal end of the resiliently deformable joint. By pulling the tendon, the resiliently deformable joint bends towards the corresponding direction. The bending joint depends purely on the dimensions of the flexural part as well as the joint's material properties, such as stiffness and yield strength. These geometrical and material properties also determine how torsion-stiff the joint is, as well as how well it can resist axial and transverse forces. These designs often involve using laser profiling techniques or electrical discharging matching techniques to create notches on a metal tube. Nitinol and other materials with lower elastic modules are often selected for these designs. The design principle is to cut out unnecessary material, thus creating clearance for the bend. Because this type of joint is designed as a combination of thin and thick sections, it often introduces stress concentration in the corner area. This means that plastic deformation and fatigue could be a major concern for long-term use. Difficulty in drilling tendon guiding channels in this type of resiliently deformable joint is another drawback. Current methods include using wire EDM to cut the channels through a small entrance or inserting tendon guiding plates through slots created on the wall of the tube. The former brings difficulties for the manufacturing and the latter brings difficulties for assembly.

### Rigid Joints

The other resiliently deformable joint category achieves bending using a plurality of small rigid joints. This type of resiliently deformable joint is built by stacking a series of small tubular segments rather than using one single continuum body. The motion between two adjacent segments is constrained by the connecting means created on the faces of the tube segments. These connecting means include rolling and hinge joints. The two parts of a rolling joint rotate with respect to each other along a circular trajectory defined by the joint's curvature, while the two parts of a hinge joint rotate with respect to a common pivot point. The connecting means are often designed as mutually fitting jigsaw puzzle pieces to limit the movement in unwanted directions. Creating tendon guiding channels is not difficult for the small tubular segments and they do not have the problem of fatigue associated with bending joints. However, friction and backlash are introduced to this design category. Due to the non-linear property of friction and backlash, this type of flexible joint has a smooth body curvature and less accurate tip positioning compared to the bending joint. In addition, compared to the unitary design of the bending joint, this type of joint introduces difficulties in assembly.

US 8,347,754 B1 discloses a system including an articulating shaft which includes a plurality of shaft sections and one or more movable joints. The one or more movable joints have a two-degree of freedom motion and operatively connect the plurality of shaft sections. The system also includes one or more cables which aid in controlling the movements of the movable joints.

US 2005/0131279 A1 discloses a video endoscope system including an articulation joint at the distal end of the endoscope which allows the distal end to be oriented by actuators. EP 0764424 A2 discloses a bending mechanism comprising a tubular body or linear body having a bending part, a movable part which can move in the lengthwise direction of said tubular body or linear body, two actuators formed to antagonize via said movable part, and a pull wire which extends in the lengthwise direction along the bending part.

### Summary of the Invention

According to a first aspect of the present invention there is provided a resiliently deformable joint having a proximal end, a distal end and an axis, the resiliently deformable joint comprising a helical structure comprising a plurality of integrally formed body portions, each of which body portions comprising a turn of the helical structure and being moveable relative to adjacent body portions, the resiliently deformable joint further comprising a plurality of body joint components, each of which body joint components is formed on a respective body portion such that adjacent body joint components are abuttable with one another to form a body joint comprising a rolling joint, the resiliently deformable joint further comprising first and second guides extending axially from the distal end to the proximal end of the resiliently deformable joint.

The use of a helical structure to form the resiliently deformable joint means that stress is more uniformly distributed along the body of the resiliently deformable joint during bending. Since a helical structure represents an assembly of an infinite number of flexure points, it can be difficult to control fully and reliably. However, the presence of the body joints results in a resiliently deformable joint which may be accurately controlled.

Adjacent body joint components will abut during bending of the resiliently deformable joint, and the contact surface of each body joint component will be curved.

In embodiments of the invention the helical structure comprises a spring and has good torsional stiffness but very low axial and bending stiffness. The body joints serve to limit the number of degrees of freedom (DOF) and also to increase the axial stiffness of the resiliently deformable joint.

The helical structure may have a substantially constant pitch along the length of the resiliently deformable joint. In other embodiments of the invention, the pitch of the helical structure may vary along the length of the resiliently deformable joint. In some embodiments of the invention the pitch of the helical structure will decrease along the length of the flexible joint.

In such embodiments of the invention, the size of the body joints may also vary with the varying pitch of the helical structure.

### Summary of the Disclosure

In embodiments of the invention where the pitch of the helical structure decreases along the length of the flexible joint, the size of the body joint components may also decrease along the length of the flexible joint.

In embodiments of the invention the resiliently deformable joint is formed from a single material. In other embodiments of the invention, the resiliently deformable joint is formed from more than material.

The particular material or materials chosen to form the resiliently deformable joint will depend on use to which a surgical instrument comprising the resiliently deformable joint is to be put.

The helical structure may comprise a single, double or multiple helix structure. When the structure comprises a double helix structure, pairs of body joint portions forming a body joint are symmetrically arranged.

For a given axial length, a double helix design has higher bending stiffness compared to a single helix design.

In general, for a given pitch distance if a helical structure uses more strands, i.e., it is a double or triple helix, the overall stiffness is increased compared with helical structures using fewer stands.

When a helical structure has an even number of strands, so for example when the helical structures are double or quadruple structures, and the starts of the stands are equally allocated, pairs of body joint portions forming a body joint will be symmetrically arranged relative to the bending plane of the flexible joint. This feature can provide an equal torsion stiffness for the structure when it is twisted either clockwise or anti-clockwise.

In contrast for a helical structure having an odd number of strands, such as a single helix structure, when that structure is twisted around its central axis, it has different torsion stiffness when twisted clockwise compared to when twisted anti-clockwise.

The particular helical structure of the resiliently deformable joint will be determined by the use to which a surgical instrument comprising the deformable joint is to be put.

A resiliently deformable joint according to embodiments of the invention comprising a plurality of body portions which are integrally formed with one another. This means that disadvantages associated with the assembly of a plurality of separate body portions are overcome.

A resiliently deformable joint according to embodiments of the invention may be bent through use of a pair of antagonist tendons. The tendons are guided through the resiliently deformable joint by means of the first and second guides.

In embodiments of the invention, the first and second guides comprise first and second channels respectively, each channel extending from the distal end to the proximal end of the resiliently deformable joint.

The channels may be formed in any convenient part of the resiliently deformable joint and serve to guide and protect the tendons during use of the resiliently deformable joint.

In embodiments of the invention, the first and second channels are radially spaced apart from the body joint portions. This is so that when bending is activated by means of antagonistic tendons extending through the first and second channels respectively, bending will take place at the body joints.

In embodiments of the invention, each body portion comprises two body joint components spaced apart radially from another by substantially 180 degrees.

The first and second guides may also be spaced apart from one another radially by 180 degrees, and in embodiments of the invention the first and second guides are radially spaced apart from each of the body joint portions by substantially 90 degrees.

Such a configuration ensures that when antagonistic tendons are in place within the first and second guides, bending may be achieved via the tendons due to the position of the first and second guides relative to the body joint portions.

The body joints may be arranged to extend in a direction that is substantially coaxial with the axis of the resiliently deformable joint. In other arrangements, the body joints may extend along a helical path.

The arrangement of the body joints will be determined by the use to which a surgical instrument comprising the resiliently deformable joint is to be put.

The first and second guides may extend generally parallel to the body joints, regardless of the arrangement of the body joints.

During bending, the body joint portions will abut with one another and move relative to one another to thus allow the bending to occur.

The body joint components may have any suitable shape to form any suitable body joint.

In embodiments of the invention, each body joint portion is substantially cylindrical in shape, with the axis of the cylinder being substantially perpendicular to the axis of the resiliently deformable joint. The contact surface of each body joint will then comprise the curved surface of the cylinder.

In embodiment of the invention the resiliently deformable joint comprises third and fourth guides, which third and fourth guides extend through a neutral bending line of the resiliently deformable joint.

In embodiments of the invention, the third and fourth guides comprises third and fourth channels respectively.

Embodiments of the invention thus provide a combination of a compliant, or resiliently deformable joint structure in the form of the helical spring, together with a rigid structure, in the form of the body joints. This results in a joint that is more flexible than known similar joints and which has its stress more uniformly distributed along its whole body when bent. In addition, due to the compliant structure of the spring, the overall elasticity becomes the dominating force influencing the bending shape, rather than local friction between body joint portions, as is the case in known similar joints. Because of this, all the body joints will present equal rotational angles, making a constant bending curvature for the resiliently deformable joint according to embodiments of the invention.

In an embodiment of the invention, the resiliently deformable joint has a diameter of approximately 9mm, and an axial length of approximately 18mm.

The resiliently deformable joint has an internal diameter of approximately 5mm.

In one embodiment of the invention the resiliently deformable joint is made from 316 stainless steel.

According to a second aspect of the present invention there is provided a resiliently deformable joint structure comprising first and second resiliently deformable joints each according to the first aspect of the present invention, wherein the first and second resiliently deformable joints are serially connected to one another with the proximal end of the first resiliently deformable joint being connected to the distal end of the second resiliently deformable joint to form a connecting portion, the connecting portion comprising first and second connecting guides which join the first and second guides of the first resiliently deformable joint with the first and second guides of the second resiliently deformable joint respectively to form first and second structure guides respectively, wherein the second resiliently deformable joint comprises third and fourth guides, which guides extend through a neutral bending line of the second resiliently deformable joint.

In embodiments of the invention, the first and second resiliently deformable joints forming the resiliently deformable joint structure are formed integrally with one another.

In embodiments of the invention the first resiliently deformable joint may be offset radially from the second resiliently deformable joint by substantially 90 degrees. Such a structure results in a spatially bendable joint through use of two pairs of tendons.

In such embodiments, the first and second guides of the first resiliently deformable joint are off set radially from the first and second guides of the second resiliently deformable joint by approximately 90 degrees and are substantially colinear with the third and fourth guides of the second resiliently deformable joint.

In such embodiments of the invention, the first and second connecting guides extend substantially axially within the connecting portion.

In other embodiments of the invention, the first resiliently deformable joint has substantially the same radial orientation as the second resiliently deformable joint. This results in a structure that is able to achieve S bending by using two pairs of tendons.

In such embodiments of the invention the first and second connecting guides extend along a substantially helical path. This enables the first and second guides of the first resiliently deformable joint to connect with the third and fourth guides of the second flexible portion respectively.

In use, therefore, tendons associated with the first resiliently deformable joint are guided to extend along a neutral bending line of the second resiliently deformable joint. In other embodiments, the first and second connecting guides may define a different path and may not be helical, but nevertheless will still follow a generally curved or stepped path in order to enable the first and second guides of the first resiliently deformable joint to join up with the third and fourth guides of the second resiliently deformable joint.

An advantage of this arrangement is that it is not necessary to use Bowden cables or the like to route the tendons, as is the case in the prior art.

In other embodiments of the second aspect of the invention the resiliently deformable joint structure may comprise more than two of resiliently deformable joints. In such embodiments of the invention the adjacent resiliently deformable joints may have the same radial orientation as one another, or one resiliently deformable joint may be rotated radially by 90 degrees relative to an adjacent radial joint. Different combinations of orientations may be present in a resiliently deformable joint structure having a plurality of resiliently deformable joints.

In such embodiments, the resiliently deformable joint structure will comprise a plurality of connecting portions connecting the distal end of one resiliently deformable joint with the proximal end of an adjacent resiliently deformable joint.

All resiliently deformable joints forming such a resiliently deformable joint structure, apart form the first resiliently deformable joint, will have third and fourth guides extending along a neutral bending line of a respective resiliently deformable joint. This is to ensure that the resiliently deformable joints forming the resiliently deformable joint structure may be decoupled from one another. In other words, by ensuring that tendons that are associated with one resiliently deformable joint extend along a neutral bending line of all other resiliently deformable joints forming the resiliently deformable joint structure, operation of those tendons will result in the bending of only the resiliently deformable joint associated with those tendons.

In some embodiments of the invention, the first resiliently deformable joint may also have third and fourth guides, even though they are not required. This could simplify the manufacturing process.

In embodiments of the invention, all guides comprise channels formed in the body portions of each resiliently deformable joint.

In embodiments of the invention the resiliently deformable joint structure further comprises a gimbal serially connected to a resiliently deformable joint.

In embodiments of the invention, the gimbal is positioned between the distal end of the resiliently deformable joint structure and the distal end of the first resiliently deformable joint.

In other embodiments of the invention, the gimbal may be positioned between two resiliently deformable joints. In yet other embodiments of the invention, the gimbal may be positioned between the proximal end of a resiliently deformable joint and the proximal end of the resiliently deformable joint structure.

According to a third aspect of the invention there is provided a surgical instrument having a proximal end and a distal end and a resiliently deformable joint in accordance with the first aspect of the invention.

According to a fourth aspect of the invention there is provide a surgical instrument having a proximal end and a distal end and a resiliently deformable joint structure in accordance with the second aspect of the invention.

The invention will now be described by way of example only with reference to the accompanying drawings in which:
Figure 1 is a schematic representation of a first embodiment of a resiliently deformable joint according to the first aspect of the invention in a neutral position;
Figure 2 is a detailed representation of a portion of the resiliently deformable joint of Figure 1 in a bent configuration;
Figure 3 is a schematic representation of a first embodiment of a resiliently deformable joint structure according to the second aspect of the invention in which the orientation of a first resiliently deformable joint is off set radially by 90 degrees relative to the second resiliently deformable joint forming the compound resiliently deformable joint;
Figure 4 is a schematic representation of a second embodiment of a resiliently deformable joint structure according to the second aspect of the invention in which the orientation of the first resiliently deformable joint is radially aligned with the second resiliently deformable joint to form an S-bending resiliently deformable joint structure;
Figure 5 is a schematic representation showing the resiliently deformable joint structure of Figure 4 in an "S" configuration;
Figure 6 is a schematic representation showing the compound resiliently deformable joint of Figure 4 in a "C" shaped configuration;
Figure 7 is a schematic representation showing a portion of another embodiment of a resiliently deformable joint according to the first aspect of the invention comprising a double helical structure;
Figure 8 is a schematic representation of a portion another embodiment of the resiliently deformable joint according to the first aspect of the invention showing a different type of rolling joint;
Figures 9 and 10 are schematic representations showing a portion of a further embodiment of the invention in which a channel has been formed in the body joint components;
Figure 11 is a schematic representation of a resiliently deformable joint according to an embodiment of the first aspect of the invention comprising a helical structure having a varying pitch along the length of the resiliently deformable joint;
Figure 12 is a schematic representation of another embodiment of a resiliently deformable joint structure according to another embodiment of the second aspect of the invention comprising three integrally formed joints;
Figure 13 is a schematic representation of a surgical instrument comprising a flexible endoscope and having a resiliently deformable joint structure according to an embodiment of the second aspect of the invention;
Figure 14 is a schematic representation of the surgical device of Figure 13 showing the joint structure with an S shaped bend;
Figure 15 is a schematic representation of the surgical instrument shown in Figures 13 and 14 showing how the tendons are routed through the instrument.

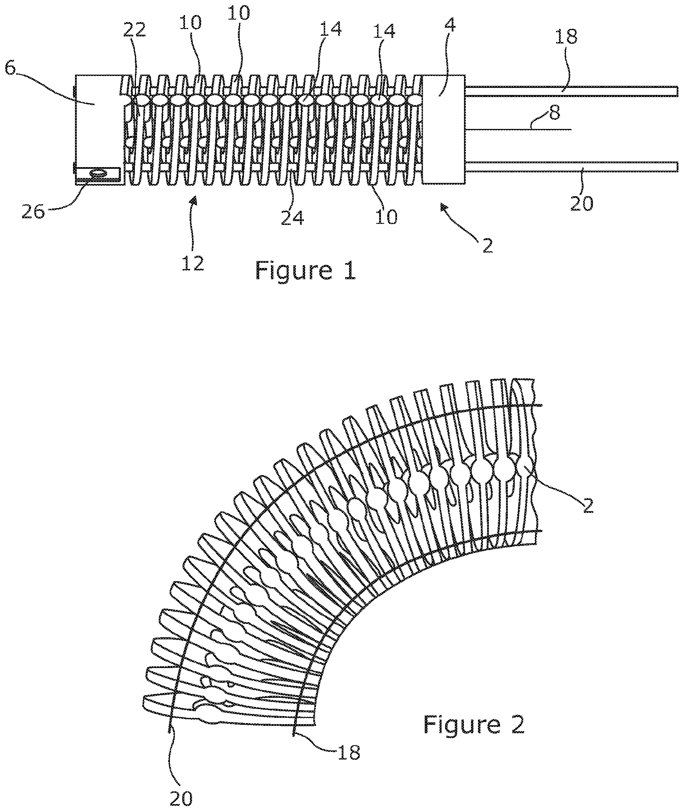

Referring first to Figure 1, a resiliently deformable joint according to an embodiment of the invention is designated generally by the reference numeral 2. The joint 2 is shown in a neutral, or unbent position in Figure 1.

The joint comprises a proximal end 4, a distal end 6 and an axis 8. The joint comprises a plurality of integrally formed body portions 10, which, in this embodiment comprise turns of a helical spring 12. The joint further comprises a plurality of body joint portions 14 formed on the body portions 10. In this embodiment each body portion, or turn of the helical spring, comprises two body joint portions 14 which are positioned diametrically opposite to one another, ie, they are radially spaced apart from one another by 180 degrees. Each body joint portion 14 is positioned to abut with a body joint portion on an adjacent body portion 10. Adjacent body joint portions 14 together form a body joint 16. In this embodiment the body joints 16 comprise rolling joints. However, in other examples not forming part of the invention, the body joints could be other joints such as hinged joints or pivot joints.

The resiliently deformable joint 2 thus comprises a bendable portion in the form of the helical spring 12, the axial compression of which is constrained by the body joints 16, which in this embodiment each comprise a rigid rolling contact joint positioned on each body portion 10. Each body joint portion 14 is cylindrical in shape, with the axis of each cylinder being substantially orthogonal to the axis 8 of the joint 2. In this embodiment, the body joints 16 are arranged in two lines extending axially which are 180 degrees apart on the surface of the resiliently deformable joint 2. Due to the constrains of the joints 16, the spring 12 has only one DOF bending which is controlled by antagonistic tendons 18, 20 as will be described in more detail below.

The resiliently deformable joint 2 further comprises first and second guides in the form of channels 22, 24 which guide the tendons 18, 20 through the joint 2. The tendons 18, 20 are attached to the resiliently deformable joint 2 at the distal end 6 by means of punch points 26 and extend through the resiliently deformable joint 2 and through the proximal end 4. The tendons 18, 20 are a pair of antagonistic tendons and can be used to control bending of the joint 2 in a known manner. In particular, by pulling on one of the tendons 18, 20, each body joint 16 is caused to deflect through a small angle. Collectively, these small angles result in the resiliently deformable joint 2 being able to bend through a large angle of up to 180 degrees or more.

Turning now to Figure 2, a portion of the resiliently deformable joint 2 is shown in a bending position. This bending position is achieved by pulling on the tendon 18.

The resiliently deformable joint 2 as shown in Figures 1 and 2 bends only within a 2D plane.

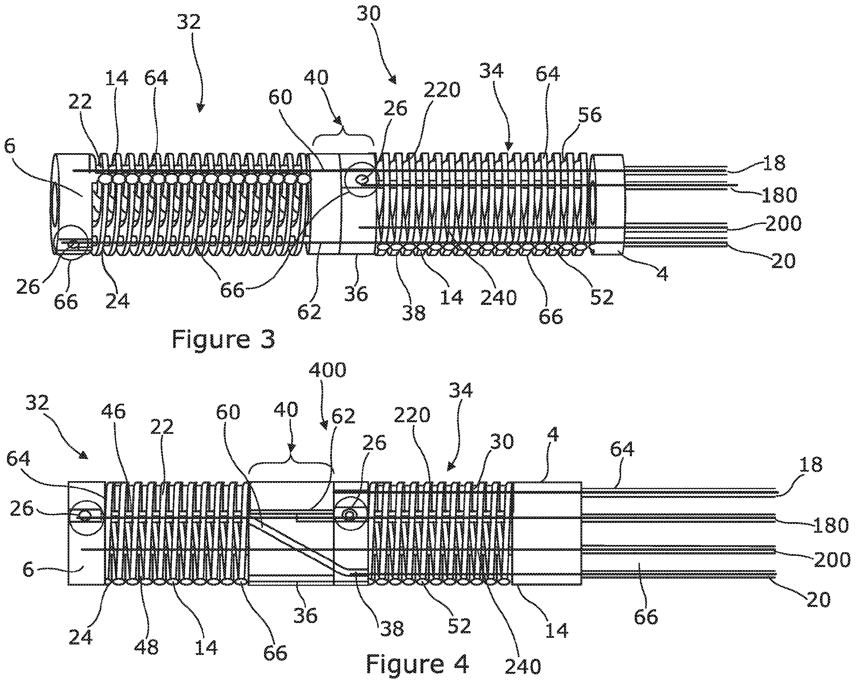

Turning now to Figure 3, an embodiment of a resiliently deformable joint structure according to the second aspect of the invention is designated by the reference numeral 30. The resiliently deformable joint structure 30 comprises a first resiliently deformable joint 32 and a second resiliently deformable joint 34 which are serially connected to one another such that the proximal end 36 of the first resiliently deformable joint 32 is connected to the distal end 38 of the second resiliently deformable joint 34 to form a connecting portion 40.

Parts of the first and second resiliently deformable joints 32, 34 that correspond to parts of the resiliently deformable joint 2 illustrated in Figure 1 have been given corresponding reference numerals for ease of reference.

The first resiliently deformable joint 32 is driven by tendons 18, 20 which are guided through the first resiliently deformable joint 32 by first and second channels 22, 24. The tendons 18, 20 are secured to the distal end 6 of the first resiliently deformable joint 32 by means of punch points 26. The second resiliently deformable joint 34 is driven by tendons 180, 200 which are guided through the second resiliently deformable joint 34 by first and second channels 220, 240, and are secured to the distal end 38 of the second resiliently deformable joint 34 by means of punch points 26. The second resiliently deformable joint 34 further comprises third and fourth guides in the form of channels 50, 52 for guiding the tendons 18, 20 through the second resiliently deformable joint 34. In order to ensure that when the tendons 18, 20 are activated in order to bend the first resiliently deformable joint 32, they do not also cause bending in the second resiliently deformable joint 34, the second resiliently deformable joint comprises third and fourth guides in the form of channels 50, 52 which extend along a neutral bending line in the second resiliently deformable joint 34. In this embodiment, the channels 50, 52 extend through the body joint portions 14 in the second resiliently deformable joint 34. This means that when the tendons 18, 20 are pulled in order to cause bending in the first resiliently deformable joint 32, they will have no effect on the second resiliently deformable joint 34. The two joints 32. 34 are therefore decoupled from one another.

The connecting portion 40 comprises first and second connecting guides in the form of channels 60, 62 which connect the channels 22, 24 in the first resiliently deformable joint with channels 50, 52 respectively to form structure channels 64, 66 extending from the distal end 6 of the first resiliently deformable joint 32 to the proximal end 4 of the second resiliently deformable joint 34, thus guiding the tendons 22, 24 from the distal end 6 of the first resiliently deformable joint 32 through both joints 32, 34.

In this embodiment of the invention, the first resiliently deformable joint 32 is radially offset from the second resiliently deformable joint 34 by substantially 90 degrees. This means that the channels 22, 24 are axially aligned with the channels 50, 52 and thus the connecting channels 60, 62 extend substantially coaxially with the axis 8 of the first and second resiliently deformable joints 32, 34.

Turning now to Figure 4, a resiliently deformable joint structure according to a second embodiment of the second aspect of the invention is designated generally by the reference numeral 400. Parts of the structure 400 that correspond to parts of the structure 30 illustrated in Figure 3 have been given corresponding reference numerals for ease of reference.

In this embodiment, the first resiliently deformable joint 32 is radially aligned with the second resiliently deformable joint 34. This means that the channels 22, 24 are axially offset from the channels 50,52 in the second resiliently deformable joint 34 and thus it is necessary for the connecting channels 60, 62 to follow a helical path in order to connect to channels 22, 24 to the channels 60, 62 to form structure channels 64, 66.

In both resiliently deformable joint structures 30, 400, the third and fourth guides are in the form of channels 50, 52 which guide the tendons 18, 20 that drive the first resiliently deformable joint 32 along a neutral bending line in the second resiliently deformable joint 34, thus decoupling the first resiliently deformable joint from the second resiliently deformable joint. This is facilitated by the connecting portion 40 which comprises connecting channels 60, 62 which guide the tendons 18, 20 from channels 18, 20 to channels 50, 52.

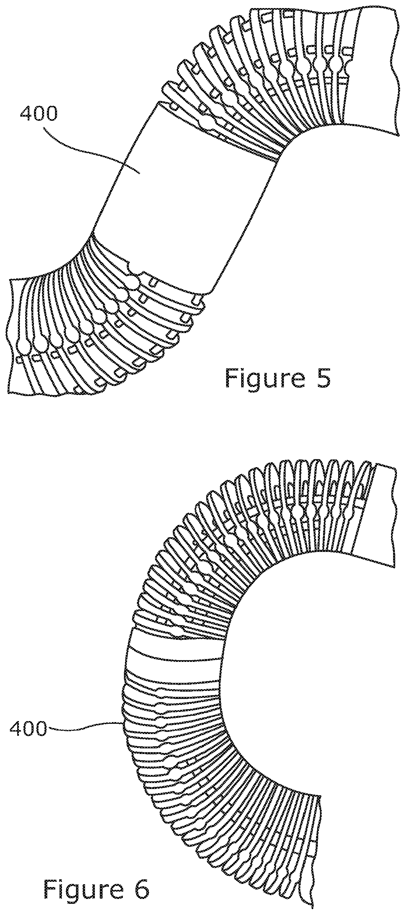

By means of the present invention it is possible to control bending through a wide range of bend shapes. Figure 5 shows the flexible structure 400 in an S bend configuration, whilst Figure 6 shows the flexible structure 400 in a C bend configuration.

In other embodiments of the invention, the flexible structure could be formed from more than two resiliently deformable joints 2. In such embodiments, adjacent joints 2 would be connected via a connection portion 40 as described above with reference to Figures 3 and 4. In such embodiments tendons used to activate a first resiliently deformable joint would be carried along a neutral bending line of each other resiliently deformable joint in order to ensure that all resiliently deformable joints were decoupled from one another.

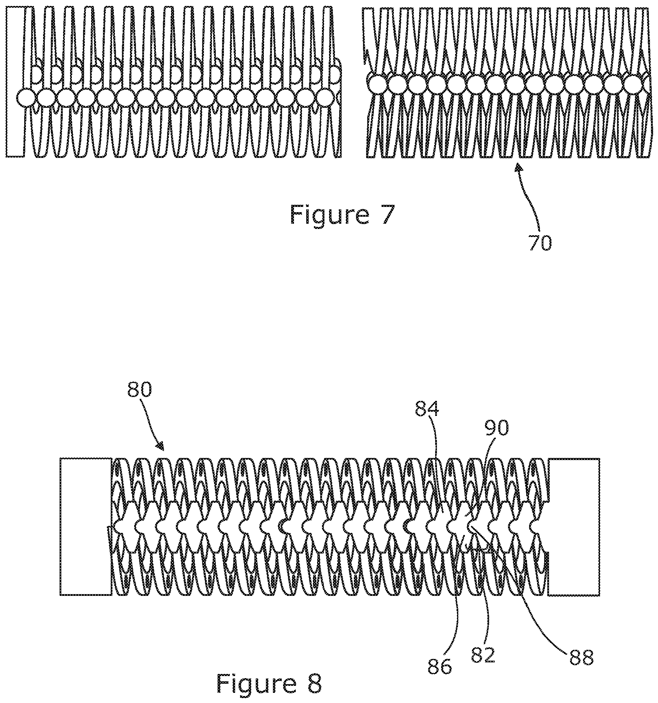

The resiliently deformable joints 2 described herein above all have a single helix structure. In other embodiments, the joint 2 could have a double or multiple helix structure. Figure 7 shows a portion of a resiliently deformable joint 70 according to another embodiment of the invention having a double helix structure.

Other forms of body joint are also possible. Figure 8 shows a portion of a resiliently deformable joint 80 according to another embodiment of the invention comprising body joints 82 formed from a first body joint portion 84 comprising a recess 86 adapted to receive a protrusion 88 forming part of a second body joint 90. The body joints 82 comprise rolling joints each of which has a circular contact surface. Such an arrangement provides more constraints on the bending motion, which also increases the torsion stiffness, although the structure requires more space than a double convex rolling joint. Other configurations are also possible.

In order to enable the tendons 18, 20 to pass along a neutral bending line, it can sometimes be necessary to pass the tendons through the middle of joints 16. In such embodiments there is a risk that the relative rigidity of the tendons may interfere with movement of the joint.

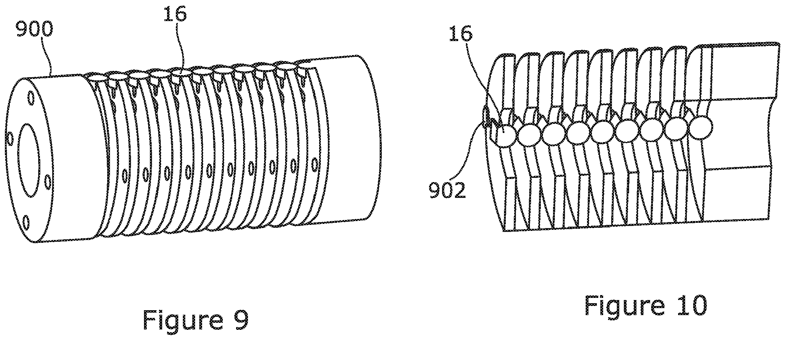

Figures 9 and 10 illustrate a resiliently deformable joint 900 according to a further embodiment of the invention in which apertures 902 have been created in the rolling joints 16. These apertures 902 may thus form the third and fourth guides which guide the tendons from another resiliently deformable joint forming part of a flexible structure according to embodiments of the invention along a neutral bending line of the joint 900.

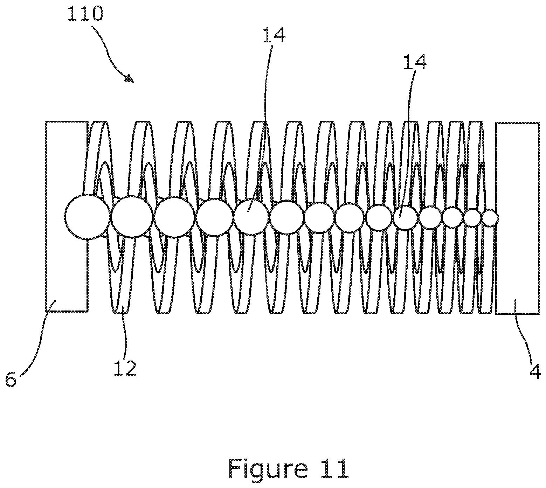

Referring now to Figure 11 a resiliently deformable joint according to another embodiment of the first aspect of the invention is designated generally by the reference numeral 110. Parts of the joint 110 which correspond to parts of the joints described hereinabove have been given corresponding reference numerals for ease of reference. In this embodiment of the invention the helical spring 12 has a pitch which increases from the proximal end 4 to the distal end 6 of the joint 110. In addition, the size of the body joint portions 14 also increases from the proximal end 4 to the distal end 6 so that the size of the body joint portions 14 varies in a corresponding manner to the variation of the pitch of the helical spring 2.

The larger the pitch of a particular portion of the helical spring 12, the greater the bending stiffness of that portion of the joint 110. In addition, a varying bending curve may be achieved. For example the distal end of the joint 110 can bend more than can the proximal end of the joint 110. This feature results in an increasing distal dexterity whilst reducing body movement of the joint 4. This is advantageous since body movement may cause damage to the surrounding environment.

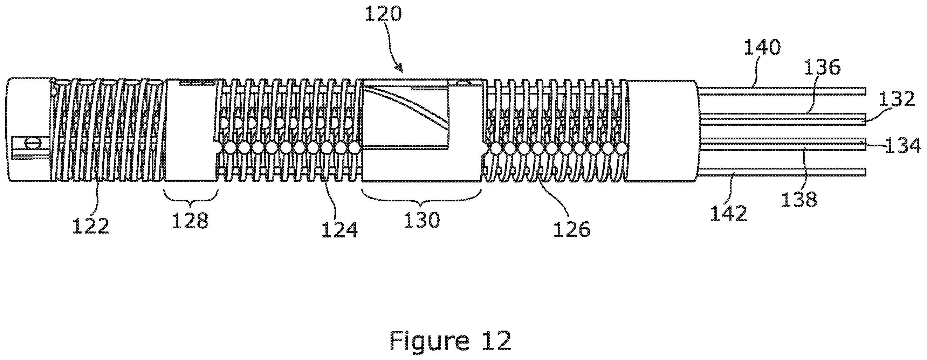

Turning now to Figure 12 a resiliently deformable joint structure according to another embodiment of the second aspect of the present invention is designated generally by the reference numeral 120.

The resiliently deformable joint structure 120 comprises first, second and third resiliently deformable joints 122, 124 and 126 which are serially connected to one another. Joint 122 is connected to joint 124 by means of connecting portion 128, and joint 124 is connected to joint 126 by means of connecting portion 130. Joint 122 is driven by tendons 132, 134 which are guided along a neutral bending line in each of the joints 124, 126. Joints 122, 124 are orientated relative to one another in the manner described hereinabove with reference to joints 32, 34 as illustrated in Figure 3 and described hereinabove. The connecting portion 128 therefore corresponds to connecting portion 40 in Figure 3.

Joints 124 and 126 are orientated relative to one another in the way illustrated in Figure 4 with respect to joints 32, 34 and connecting portion 130 is of the same type as connection portion 40 shown in Figure 4.

Joint 124 is controlled by tendons 136 and 138, and these tendons are guided along neutral bending lines in joint 126. Joint 126 is controlled by tendons 140, 142.

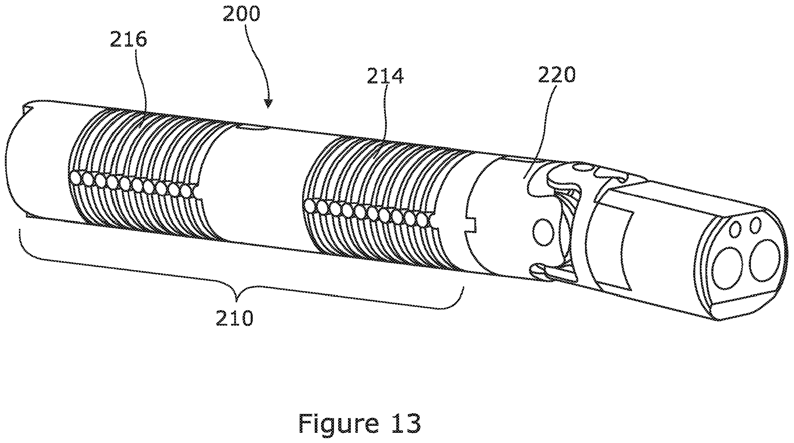

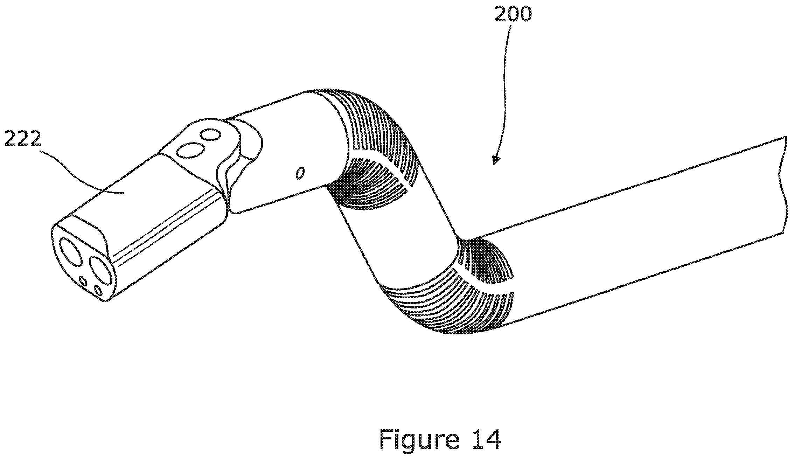

Turning now to Figures 13 and 14 a surgical instrument comprising a flexible endoscope is designated generally by the reference numeral 200.

The endoscope 200 comprises a resiliently deformable joint structure 210 of the type illustrated in Figure 4 and described hereinabove. This joint structure 210 is therefore adapted to be able to form an "S" when bent, as shown in Figure 14.

The flexible endoscope 200 further comprises a gimbal serially connected to joint 214 forming part of the joint structure 210.

An endoscope of this type could be used for endoluminal surgery and in a single port surgical system.

The joint structure 210 allows for S shape bending as shown in Figure 14. The gimbal 220 has two degrees of freedom.

For a single port surgical system, all surgical instruments including a flexible endoscope such as flexible endoscope 210 are passed through a single surgical port. The flexible endoscope comprises a camera 222 which is required to assume an elevated position in order to look down on an area to be operated on. This is to allow an optimal view of the surgical field.

By means of the present invention these requirements may be fulfilled.

By means of the joint structure 210 the position of the camera may be raised relative to the remainder of the surgical instrument. The gimbal 220 is adapted to perform tilt and pan movement for the camera.

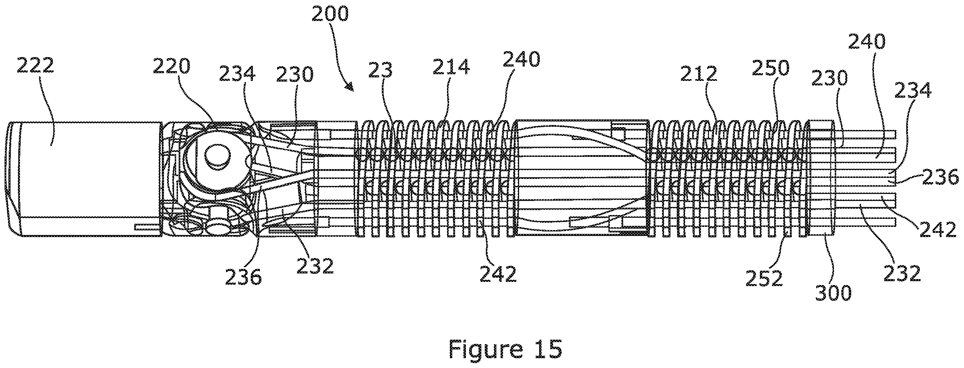

Turning now to Figure 15, the routing of the tendons used to control the endoscope 200 is shown in more detail. In a similar manner to that described hereinabove with reference to the other embodiments of the invention, all tendons used to control either the gimbal 220 or the joint 214, when passing through the other one or more joints 214, 212 respectively, are passed through neutral bending lines of those other joints.

In this example, the gimbal is controlled by tendons 230, 232, 234 and 236. These tendons pass through neutral bending lines of joints 212 and 214 before emerging beyond the proximal end 300 of the surgical instrument 200.

Similarly joint 214 is controlled by tendons 240, 242 which are guided along neutral bending lines in joint 212 before emerging from the proximal end 300 of the device 200.

Finally, joint 212 is controlled by tendons 250, 252 in the manner described hereinabove with reference to the other embodiments of the invention.

The resulting flexible endoscope 200 benefits from the fact that the position and orientation of the camera 222 are decoupled from the remaining joints 212, 214. This facilitates control of the device 200 particularly when a user wishes to control only the camera.

By controlling the bending of the joints 212, 214, the position of the camera 222 may be changed without changing the viewing angle of the camera.

## Claims

1. A resiliently deformable joint (2) having a proximal end (4), a distal end (6) and an axis (8), the resiliently deformable joint (2) comprising a helical structure comprising a plurality of integrally formed body portions (10), each of which body portions (10) comprising a turn of the helical structure and being moveable relative to adjacent body portions (10), the resiliently deformable joint (2) further comprising a plurality of body joint components, each of which body joint components is formed on a respective body portion such that adjacent body joint components are abuttable with one another to form a body joint (16), the resiliently deformable joint (2) further comprising first and second guides extending axially from the distal end (6) to the proximal end (4) of the resiliently deformable joint (2);
**characterised in that** the body joint (16) comprises a rolling joint.

2. A resiliently deformable joint (2) as claimed in claim 1 wherein the pitch of the helical structure varies along the length of the resiliently deformable joint (2) and, optionally, the size of the body joints (16) varies with the varying pitch of the helical structure.

3. A resiliently deformable joint (2) as claimed in claim 1 or claim 2 wherein the first and second guides comprise first and second channels (22, 24) respectively, each channel extending from the distal end (6) to the proximal end (4) of the resiliently deformable joint (2); optionally, each body portion (10) comprises two body joint components radially spaced apart from one another by substantially 180 degrees; and, further optionally, the first and second channels (22, 24) are spaced apart radially from each of the body joint portions by substantially 90 degrees.

4. A resiliently deformable joint (2) as claimed in any one of the preceding claims wherein each body joint portion is cylindrical, with the axis of the body joint portion being substantially perpendicular to the axis of the resiliently deformable joint (2).

5. A resiliently deformable joint (2) as claimed in any one of the preceding claims further comprises third and fourth guides, which guides extend through a neutral bending line of the resiliently deformable joint (2) and, optionally, the third and fourth guides comprise third and fourth channels (50, 52) respectively.

6. A resiliently deformable joint structure (30) comprising first and second resiliently deformable joints (32, 34) as claimed in any one of claims 1 - 5 wherein the first and second resiliently deformable joints (32, 34) are serially connected to one another with the proximal end (36) of the first resiliently deformable joint (32) being connected to the distal end (38) of the second resiliently deformable joint (34) to form a connecting portion (40), the connecting portion (40) comprising first and second connecting guides which connect the first and second guides of the first flexible portion with the first and second guides of the second flexible portion respectively to form first and second structure guides respectively, which first and second structure guides extend from the distal end (38) of the first resiliently deformable joint (32) to the proximal end (36) of the second resiliently deformable joint (34), and wherein the second resiliently deformable joint (34) comprises third and fourth guides which guides extend through a neutral bending line of the second resiliently deformable joint (34).

7. A resiliently deformable joint structure (30) as claimed in claim 6 wherein the first resiliently deformable joint (32) is offset from the second resiliently deformable joint (34) radially by 90 degrees.

8. A resiliently deformable joint structure (30) as claimed in claim 7 wherein the first and second guides of the first resiliently deformable joint (32) are offset radially from the first and second guides of the second resiliently deformable joint (34) by approximately 90 degrees, and wherein the first and second guides of the first resiliently deformable joint (32) are substantially colinear with the third and fourth guides of the second resiliently deformable joint (34) respectively.

9. A resiliently deformable joint structure (30) as claimed in claim 7 or claim 8 wherein the first and second connecting guides extend substantially axially.

10. A resiliently deformable joint structure (30) as claimed in any one of claims 6 to 9 wherein the first resiliently deformable joint (32) is axially aligned with the second resiliently deformable joint (34) and, optionally, the first and second connecting guides extend helically within the connecting portion (40).

11. A resiliently deformable joint structure (30) as claimed in any one of claims 6 to 10 comprising more than two resiliently deformable joints, and a plurality of connecting portions connecting the distal end of each of which connecting portions connects the distal end of one resiliently deformable joint with the proximal end of an adjacent resiliently deformable joint.

12. A resiliently deformable joint structure (30) as claimed in any one of claims 6 to 11 wherein the first resiliently deformable joint (32) comprises third and fourth guides extending through neutral bending lines of the first resiliently deformable joint (32).

13. A resiliently deformable joint structure (30) according to any one of claims 6 to 12 further comprising a gimbal serially connected to a resiliently deformable joint.

14. A surgical instrument comprising a proximal end and a distal end and a resiliently deformable joint (2) according to any one of claims 1 to 5 positioned at the distal end of the instrument.

15. A surgical instrument comprising a proximal end and a distal end and a resiliently deformable joint structure (30) according to any one of claims 6 to 13 positioned at the distal end of the instrument.

## Patentansprüche

1. Elastisch verformbares Gelenk (2) mit einem proximalen Ende (4), einem distalen Ende (6) und einer Achse (8), wobei das elastisch verformbare Gelenk (2) eine spiralförmige Struktur aufweist, die mehrere integral geformte Körperabschnitte (10) umfasst, wobei jeder dieser Körperabschnitte (10) eine Windung der helikalen Struktur aufweist und relativ zu benachbarten Körperabschnitten (10) beweglich ist, wobei das elastisch verformbare Gelenk (2) weiterhin mehrere Körpergelenkkomponenten umfasst, wobei jede dieser Körpergelenkkomponenten an einem jeweiligen Körperabschnitt ausgebildet ist, so dass benachbarte Körpergelenkkomponenten aneinander stoßbar sind, um ein Körpergelenk (16) zu bilden, wobei das elastisch verformbare Gelenk (2) weiterhin erste und zweite Führungen umfasst, die sich axial vom distalen Ende (6) zum proximalen Ende (4) des elastisch verformbaren Gelenks (2) erstrecken;
**dadurch gekennzeichnet, dass** das Körpergelenk (16) ein Rollgelenk umfasst.

2. Elastisch verformbares Gelenk (2) nach Anspruch 1, wobei die Steigung der helikalen Struktur entlang der Länge des elastisch verformbaren Gelenks (2) variiert und optional die Größe der Körpergelenke (16) mit der variierenden Steigung der spiralförmigen Struktur variiert.

3. Elastisch verformbares Gelenk (2) nach Anspruch 1 oder Anspruch 2, wobei die ersten und zweiten Führungen jeweils erste und zweite Kanäle (22, 24) umfassen, sich jeder Kanal vom distalen Ende (6) zum proximalen Ende (4) des elastisch verformbaren Gelenks (2) erstreckt; optional jeder Körperabschnitt (10) zwei Körperverbindungskomponenten umfasst, die radial im Wesentlichen um 180 Grad voneinander beabstandet sind; und weiter optional die ersten und zweiten Kanäle (22, 24) radial von jedem der Körperverbindungsabschnitte um im Wesentlichen 90 Grad beabstandet sind.

4. Elastisch verformbares Gelenk (2) nach einem der vorhergehenden Ansprüche, wobei jeder Körperverbindungsabschnitt zylindrisch ist, wobei die Achse des Körperverbindungsabschnitts im Wesentlichen senkrecht zur Achse des elastisch verformbaren Gelenks (2) verläuft.

5. Elastisch verformbares Gelenk (2) nach einem der vorhergehenden Ansprüche weiterhin dritte und vierte Führungen umfasst, wobei die Führungen durch eine neutrale Biegelinie des elastisch verformbaren Gelenks (2) verlaufen und optional die dritten und vierten Führungen dritte bzw. vierte Kanäle (50, 52) umfassen.

6. Elastisch verformbare Gelenkstruktur (30), die erste und zweite elastisch verformbare Gelenke (32, 34) nach einem der Ansprüche 1 bis 5 umfasst, wobei die ersten und zweiten elastisch verformbaren Gelenke (32, 34) seriell miteinander verbunden sind, wobei das proximale Ende (36) des ersten elastisch verformbaren Gelenks (32) mit dem distalen Ende (38) des zweiten elastisch verformbaren Gelenks (34) verbunden ist, um einen Verbindungsabschnitt (40) zu bilden, wobei der Verbindungsabschnitt (40) erste und zweite Verbindungsführungen umfasst, die die ersten und zweiten Führungen des ersten flexiblen Abschnitts mit den ersten und zweiten Führungen des zweiten flexiblen Abschnitts verbinden, um jeweils erste und zweite Strukturführungen zu bilden, wobei sich erste und zweite Strukturführungen vom distalen Ende (38) des ersten elastisch verformbaren Gelenks (32) zum proximalen Ende (36) des zweiten elastisch verformbaren Gelenks (34) erstrecken, und wobei das zweite elastisch verformbare Gelenk (34) dritte und vierte Führungen umfasst, wobei sich die Führungen durch eine neutrale Biegelinie des zweiten elastisch verformbaren Gelenks (34) erstrecken.

7. Elastisch verformbare Gelenkstruktur (30) nach Anspruch 6, wobei das erste elastisch verformbare Gelenk (32) gegenüber dem zweiten elastisch verformbaren Gelenk (34) radial um 90 Grad versetzt ist.

8. Elastisch verformbare Gelenkstruktur (30) nach Anspruch 7, wobei die ersten und zweiten Führungen des ersten elastisch verformbaren Gelenks (32) radial zu den ersten und zweiten Führungen des zweiten elastisch verformbaren Gelenks (34) um etwa 90 Grad versetzt sind, und wobei die ersten und zweiten Führungen des ersten elastisch verformbaren Gelenks (32) im Wesentlichen kolinear mit den dritten bzw. vierten Führungen des zweiten elastisch verformbaren Gelenks (34) sind.

9. Elastisch verformbare gelenkstruktur (30) nach Anspruch 7 oder Anspruch 8, wobei sich die ersten und zweiten Verbindungsführungen im Wesentlichen axial erstrecken.

10. Elastisch verformbare Gelenkstruktur (30) nach einem der Ansprüche 6 bis 9, wobei die erste elastisch verformbare Verbindung (32) axial mit der zweiten elastisch verformbaren Verbindung (34) ausgerichtet ist und optional die ersten und zweiten Verbindungsführungen spiralförmig innerhalb des Verbindungsabschnitts (40) verlaufen.

11. Elastisch verformbare Gelenkstruktur (30) nach einem der Ansprüche 6 bis 10, umfassend mehr als zwei elastisch verformbare Gelenke und mehrere Verbindungsabschnitte, die das distale Ende jedes dieser Verbindungsabschnitte verbinden, das distale Ende eines elastisch verformbaren Gelenks mit dem proximalen Ende eines benachbarten elastisch verformbaren Gelenks verbinden.

12. Elastisch verformbare Gelenkstruktur (30) nach einem der Ansprüche 6 bis 11, wobei das erste elastisch verformbare Gelenk (32) dritte und vierte Führungen umfasst, die sich durch neutrale Biegelinien des ersten elastisch verformbaren Gelenks (32) erstrecken.

13. Elastisch verformbare Gelenkstruktur (30) nach einem der Ansprüche 6 bis 12, die weiterhin einen Kardanring umfasst, der in Reihe mit einem elastisch verformbaren Gelenk verbunden ist.

14. Chirurgisches Instrument, umfassend ein proximales Ende und ein distales Ende sowie ein elastisch verformbares Gelenk (2) nach einem der Ansprüche 1 bis 5, das am distalen Ende des Instruments positioniert ist.

15. Chirurgisches Instrument, umfassend ein proximales Ende und ein distales Ende sowie eine elastisch verformbare Gelenkstruktur (30) nach einem der Ansprüche 6 bis 13, die am distalen Ende des Instruments positioniert ist.

## Revendications

1. Articulation élastiquement déformable (2) ayant une extrémité proximale (4), une extrémité distale (6) et un axe (8), l'articulation élastiquement déformable (2) comprenant une structure hélicoïdale comprenant une pluralité de parties de corps formées d'un seul tenant (10), chacune de ces parties de corps (10) comprenant une spire de la structure hélicoïdale et étant mobile par rapport à des parties de corps adjacentes (10), l'articulation élastiquement déformable (2) comprenant en outre une pluralité de composants d'articulation de corps, chacun de ces composants d'articulation de corps est formé sur une partie de corps respective de telle sorte que des composants d'articulation de corps adjacents puissent venir en butée les uns avec les autres pour former une articulation de corps (16), l'articulation élastiquement déformable (2) comprenant en outre des premier et deuxième guides s'étendant axialement de l'extrémité distale (6) jusqu'à l'extrémité proximale (4) de l'articulation élastiquement déformable (2) ;
**caractérisé en ce que** l'articulation de corps (16) comprend une articulation roulante.

2. Articulation élastiquement déformable (2) selon la revendication 1, dans laquelle le pas de la structure hélicoïdale varie sur la longueur de l'articulation élastiquement déformable (2) et, éventuellement, la taille des articulations de corps (16) varie avec le pas variable de la structure hélicoïdale.

3. Articulation élastiquement déformable (2) selon la revendication 1 ou la revendication 2, dans laquelle les premier et deuxième guides comprennent respectivement des premier et deuxième canaux (22, 24), chaque canal s'étendant de l'extrémité distale (6) à l'extrémité proximale (4) de l'articulation élastiquement déformable (2) ; éventuellement, chaque partie de corps (10) comprend deux composants d'articulation de corps espacés radialement l'un de l'autre de sensiblement 180 degrés ; et, en outre éventuellement, les premier et deuxième canaux (22, 24) sont espacés radialement de chacune des parties d'articulation de corps de sensiblement 90 degrés.

4. Articulation élastiquement déformable (2) selon l'une quelconque des revendications précédentes, dans laquelle chaque partie d'articulation de corps est cylindrique, l'axe de la partie d'articulation de corps étant sensiblement perpendiculaire à l'axe de l'articulation élastiquement déformable (2).

5. Articulation élastiquement déformable (2) selon l'une quelconque des revendications précédentes, comprend en outre des troisième et quatrième guides, lesquels guides s'étendent à travers une ligne de courbure neutre de l'articulation élastiquement déformable (2) et, éventuellement, les troisième et quatrième guides comprennent respectivement des troisièmes et quatrièmes canaux (50, 52).

6. Structure d'articulation élastiquement déformable (30) comprenant des première et seconde articulations élastiquement déformables (32, 34) selon l'une quelconque des revendications 1 à 5, dans laquelle les première et seconde articulations élastiquement déformables (32, 34) sont reliées en série l'une à l'autre, l'extrémité proximale (36) de la première articulation élastiquement déformable (32) étant reliée à l'extrémité distale (38) de la seconde articulation élastiquement déformable (34) pour former une partie de liaison (40), la partie de liaison (40) comprenant des premier et deuxième guides de liaison qui relient les premier et deuxième guides de la première partie flexible aux premier et deuxième guides de la seconde partie flexible respectivement pour former les premier et deuxième guides de structure, lesquels premiers et deuxième guides de structure s'étendent de l'extrémité distale (38) de la première articulation élastiquement déformable (32) à l'extrémité proximale (36) de la seconde articulation élastiquement déformable (34), et dans laquelle la seconde articulation élastiquement déformable (34) comprend des troisième et quatrième guides qui s'étendent à travers une ligne de courbure neutre de la seconde articulation élastiquement déformable (34).

7. Structure d'articulation élastiquement déformable (30) selon la revendication 6, dans laquelle la première articulation élastiquement déformable (32) est décalée de la seconde articulation élastiquement déformable (34) radialement de 90 degrés.

8. Structure d'articulation élastiquement déformable (30) selon la revendication 7, dans laquelle les premier et deuxième guides de la première articulation élastiquement déformable (32) sont décalés radialement des premier et deuxième guides de la seconde articulation élastiquement déformable (34) d'environ 90 degrés, et dans laquelle les premier et deuxième guides de la première articulation élastiquement déformable (32) sont sensiblement colinéaires avec les troisième et quatrième guides de la seconde articulation élastiquement déformable (34), respectivement.

9. Structure d'articulation élastiquement déformable (30) selon la revendication 7 ou la revendication 8, dans laquelle les premier et deuxième guides de liaison s'étendent sensiblement axialement.

10. Structure d'articulation élastiquement déformable (30) selon l'une quelconque des revendications 6 à 9, dans laquelle la première articulation élastiquement déformable (32) est alignée axialement avec la seconde articulation élastiquement déformable (34) et, éventuellement, les premier et deuxième guides de liaison s'étendent de manière hélicoïdale à l'intérieur de la partie de liaison (40).

11. Structure d'articulation élastiquement déformable (30) selon l'une quelconque des revendications 6 à 10, comprenant plus de deux articulations élastiquement déformables, et une pluralité de parties de liaison, reliant l'extrémité distale de chacune des parties de liaison, relient l'extrémité distale d'une articulation élastiquement déformable à l'extrémité proximale d'une articulation élastiquement déformable adjacente.

12. Structure d'articulation élastiquement déformable (30) selon l'une quelconque des revendications 6 à 11, dans laquelle la première articulation élastiquement déformable (32) comprend des troisième et quatrième guides s'étendant à travers des lignes de courbure neutres de la première articulation élastiquement déformable (32).

13. Structure d'articulation élastiquement déformable (30) selon l'une quelconque des revendications 6 à 12, comprenant en outre un cardan relié en série à une articulation élastiquement déformable.

14. Instrument chirurgical comprenant une extrémité proximale et une extrémité distale et une articulation élastiquement déformable (2) selon l'une quelconque des revendications 1 à 5 positionnée à l'extrémité distale de l'instrument.

15. Instrument chirurgical comprenant une extrémité proximale et une extrémité distale et une structure d'articulation élastiquement déformable (30) selon l'une quelconque des revendications 6 à 13 positionnée à l'extrémité distale de l'instrument.
